# EUROPÄISCHE PATENTSCHRIFT

(11) **EP 1 713 589 B1**
(45) Veröffentlichungstag und Bekanntmachung des Hinweises auf die Patenterteilung: **15.09.2010**
(21) Anmeldenummer: 05701309.6
(22) Anmeldetag: 02.02.2005
(51) Int. Cl.: B01L 99/00, G01N 33/80

(54) **TESTELEMENT UND VERFAHREN ZUM TESTEN VON BLUT**
TEST ELEMENT AND METHOD FOR TESTING BLOOD
ELEMENT D'ANALYSE ET PROCEDE POUR ANALYSER LE SANG

(30) Priorität: 02.02.2004 DE 102004005139
(43) Veröffentlichungstag der Anmeldung: 25.10.2006
(73) Patentinhaber: Medion Diagnostics AG, 3186 Düdingen (CH)
(72) Erfinder: SCHWIND, Peter, CH-1700 Fribourg (CH)
(74) Vertreter: Heselberger, Johannes
(86) Internationale Anmeldenummer: PCT/EP2005/001027
(87) Internationale Veröffentlichungsnummer: WO 2005/072876

(56) Entgegenhaltungen:
- US-A- 3 905 772
- US-A- 4 650 662

## Beschreibung

Die Erfindung betrifft ein Testelement und ein Verfahren für diagnostische Tests, insbesondere zum Testen von Konserven- und Empfängerblut vor einer Bluttransfusion.

Eines der größten Risiken bei Transfusionen von Blutbestandteilen, sogenannten Bluttransfusionen ist eine Blutgruppeninkompatibilität zwischen Konserven- und Empfängerblut. Die Ursache hierfür sind häufiger Verwechslungen als Fehlbestimmungen. Aus diesem Grunde sind in einigen Ländern sogenannte ABO-Identitätstests vorgeschrieben, die durch das behandelnde Personal, beispielsweise die Krankenschwester oder den transfundierenden Arzt, am Patientenbett direkt vor der Transfusion durchgeführt werden. Diese Tests führen zu einer zusätzlichen Belastung des in Labordiagnostik wenig geschulten Stationspersonals und werden unter anderem deshalb in einigen Ländern abgelehnt.

In bestimmten Ländern wie Deutschland und Österreich ist ein solcher Identitätstest vorgeschrieben, allerdings nur bezüglich des Empfängerbluts. In diesen Ländern ist es der jeweiligen Klinik überlassen, ob sie den Identitätstest der Konserve am Patientenbett durchführen lässt oder nicht. Begründet wird dies mit der Verantwortung des Produzenten (Blutbank) für die korrekte Bestimmung und Kennzeichnung des Konservenblutes. Dies hindert jedoch viele Krankenhäuser nicht daran, die Konservenblutgruppe im Krankenhauslabor trotzdem noch einmal zu kontrollieren und/oder einen ABO-Identitätstest am Patientenbett durchführen zu lassen.

Die Aufgabe der vorliegenden Erfindung ist es, ohne Erhöhung des Aufwands das Verwechslungsrisiko bei einer Bluttransfusion nahezu auszuschließen. Darüber hinaus sollen sich die Kosten für die Bluttransfusion dadurch nicht erhöhen.

Erfindungsgemäß wird die Aufgabe durch ein Testelement für diagnostische Tests und ein Verfahren zum Testen im Rahmen der Vorbereitung und Durchführung von Bluttransfusionen gelöst, wie sie in den unabhängigen Ansprüchen beschrieben sind. Das erfindungsgemäße Testelement für diagnostische Tests, insbesondere zum Testen von Blut vor einer Bluttransfusion, weist mindestens zwei Testeinheiten zum Durchführen von je mindestens zwei Tests auf. Darüber hinaus weist das Testelement ein Befestigungsmittel zur Befestigung des Testelements auf Vorzugsweise ist das Befestigungselement derart gestaltet, dass das Testelement an einer Blutkonserve befestigt werden kann.

Mit Hilfe eines solchen Testelements kann die Gefahr einer Verwechslung einer Blutkonserve und damit die Verwendung von Blut mit nicht kompatibler Blutgruppe im Rahmen einer Bluttransfusion nahezu ausgeschlossen werden. Vorzugsweise wird mit Hilfe einer der mindestens zwei Testeinheiten des Testelements das Konservenblut für die Bluttransfusion, also das Blut eines Segments der Blutkonserve getestet. Dabei ist das Testelement derart ausgestaltet, dass das Ergebnis des Tests nach kurzer Zeit ohne zusätzliche Hilfsmittel leicht ablesbar ist.

Mit dem erfindungsgemäßen Befestigungselement kann das komplette Testelement an der entsprechenden Blutkonserve befestigt werden. Hierdurch kann jeder sehen, dass an dieser Blutkonserve ein bestätigender Blutgruppentest durchgeführt wurde, und welches Ergebnis dieser Blutgruppentest liefert. Darüber hinaus kann durch die Verwendung des erfindungsgemäßen Testelements der Bestätigungstest mit wenigen Handgriffen und in kurzer Zeit durchgeführt werden. Außerdem wird mit dem erfindungsgemäßen Testelement noch der Vorteil erzielt, dass weitere Fehler, wie beispielsweise Schreibfehler, nahezu ausgeschlossen sind.

In bevorzugten Ausführungsformen der Erfindung werden als Befestigungselement Klebefolie oder Kabelbinder verwendet.

Die zweite erfindungsgemäße Testeinheit des Testelements wird vorzugsweise verwendet, um die Gefahr der Verwendung einer Blutkonserve mit unpassender Blutgruppe weiter zu verringern. Hierfür wird mit Hilfe der zweiten Testeinheit des Testelements das Blut des Empfängers der Bluttransfusion vorzugsweise unmittelbar vor der Transfusion getestet. Die dafür notwendigen Hilfsmittel, nämlich das Testelement, wird physisch mit der Blutkonserve verbunden und damit zwangsläufig an das Patientenbett geliefert.

Vorzugsweise sind die beiden Testeinheiten des Testelements so angeordnet, dass nach der Durchführung der beiden Tests einfach zu sehen ist, ob die Blutgruppe der Blutkonserve mit der Blutgruppe des Empfängers übereinstimmt oder nicht. Dies wird vorzugsweise durch eine spiegelbildliche Anordnung der Testkammern - für flüssige Nachweisreagenzien - oder der Testfelder - für immobilisierte Nachweisreagenzien - der Testeinheiten erzielt.

In einer weiter bevorzugten Ausführungsform bleibt bei mindestens einer Testeinheit das Testergebnis in dem Test so lange bestehen und damit sichtbar, wie die Konserve gemäss Herstellerangabe haltbar ist, beispielsweise 45 Tage, so dass das Testelement auch für Protokollzwecke und zur Kontrolle verwendet werden kann. Die Anzeige des Testergebnisses, insbesondere das Testergebnis bezüglich der Blutgruppe in der Blutkonserve, bleibt vorzugsweise während der Haltbarkeitsdauer, beispielsweise 45 Tage, bei Lagerung bei 2°C bis 8°C erkennbar, damit die getestete Blutkonserve solange in einem Konservenkühlschrank aufbewahrt werden kann, bevor sie für die Bluttransfusion verwendet wird. Bei Verwendung eines Flüssigreagenz als Nachweisreagenz kann diese Haltbarkeit beispielsweise dadurch erreicht werden, dass dem Flüssigreagenz Zellstabilisatoren zugesetzt werden.

Falls flüssige Nachweisreagenzien verwendet werden sollen, ist in einer weiteren bevorzugten Ausführungsform mindestens eine der Testeinheiten zur Durchführung der Tests derart gestaltet, dass die Testkammer zur Aufnahme des Nachweisreagenz verschlossen oder verschließbar ist und nach der Durchführung des Tests keine Flüssigkeit hieraus austritt, etwa durch Verdunstung, so dass im Falle von Reaktionen in der Flüssigphase die Testeinheit nicht austrocknet und dadurch der am Patienten durchgeführte Test mit dem an der Blutkonserve durchgeführten Test später verglichen werden kann. Hierfür können beispielsweise geeignete Verschließmechanismen verwendet werden.

In einer weiteren bevorzugten Ausführungsform weist die Testeinheit für das Konservenblut mindestens drei Testkammern bzw. Testfelder auf, in denen jeweils ein Anti-A-, ein Anti-B- und ein Anti-D-Reagenz enthalten ist. Mit Hilfe dieser mindestens drei Testkammern bzw. Testfelder kann dementsprechend ein ABD-Test durchgeführt werden. In einer weiteren bevorzugten Ausführungsform ist eine weitere Testkammer bzw. ein weiteres Testfeld zur Durchführung einer Eigenkontrolle vorgesehen. Die Testeinheit für das Blut des Empfängers weist vorzugsweise mindestens zwei Testkammern bzw. Testfelder auf, in denen vorzugsweise jeweils ein Anti-A- und ein Anti-B-Reagenz enthalten ist. Mit Hilfe dieser mindestens zwei Testkammern bzw. Testfelder kann ein ABO-Test durchgeführt werden.

Erfindungsgemäß wird die Aufgabe auch durch ein Verfahren zum Testen von Blut im Rahmen der Vorbereitung und Durchführung von Bluttransfusionen gelöst, wobei das Verfahren die folgenden Schritte aufweist:
- Testen des Konservenbluts mit Hilfe einer ersten Testeinheit in einem Testelement, wie oben beschrieben, vorzugsweise im Krankenhauslabor,
- Befestigen des Testelements auf der das Konservenblut enthaltenen Blutkonserve mit Hilfe eines Befestigungsmittels, und
- Testen des Bluts des Empfängers mit Hilfe einer zweiten Testeinheit des Testelements, vorzugsweise am Patientenbett, insbesondere innerhalb von 45 Tagen nach Testen des Konservenbluts.

Das erfindungsgemäße Verfahren zum Testen von Blut hat den Vorteil, dass eine Verwendung einer Blutkonserve mit einer für den Patienten inkompatiblen Blutgruppe im Rahmen einer Bluttransfusion nahezu ausgeschlossen werden kann. Durch die Verwendung eines Testelements, das an der Blutkonserve befestigt werden kann, zum Testen des Konservenbluts und des Bluts des Empfängers wird eine Verwechslung praktisch unmöglich gemacht, denn es ist klar ersichtlich, welche Tests für die Bluttransfusion bei der diese Blutkonserve verwendet werden soll, bereits durchgeführt wurden und was das Ergebnis des jeweiligen Tests war. Die in diagnostischen Tests eher unerfahrene Krankenschwester hat durch das für sie vor Ort einsehbare reale Ergebnis des Labortests ein Referenzergebnis, das ihr die Beurteilung erleichtert, ob ihr eigenes Ergebnis korrekt ist. Dies erspart zeitraubende Rückfragen beim Krankenhauslabor.

Hinzu kommt, dass die Krankenschwester durch Blutkonserventestung im Labor wesentlich entlastet wird. Außerdem ermöglicht das erfindungsgemäße Verfahren, dass die Blutkonserven eindeutig gekennzeichnet sind und damit keine Protokolle eingesehen werden müssen.

Vorzugsweise wird dieses Verfahren verwendet, um Blutgruppen zu bestimmen. Weiter bevorzugt wird vor der Durchführung der Bluttransfusion überprüft, dass beim Testen des Konservenbluts und beim Testen des Bluts des Empfängers die gleiche Blutgruppe festgestellt wurde.

Im Folgenden wird ein Ausführungsbeispiel der Erfindung anhand der beigefügten Zeichnungen erläutert, in denen
- Figur 1: eine Draufsicht auf eine bevorzugte Ausführungsform eines erfin- dungsgemäßen Testelements zeigt,
- Figur 2: eine Draufsicht auf eine weitere bevorzugte Ausführungsform eines erfindungsgemäßen Testelements zeigt, und
- Figur 3: ein Beispiel für die Befestigung des Testelements an einer Blutkon- serve darstellt.

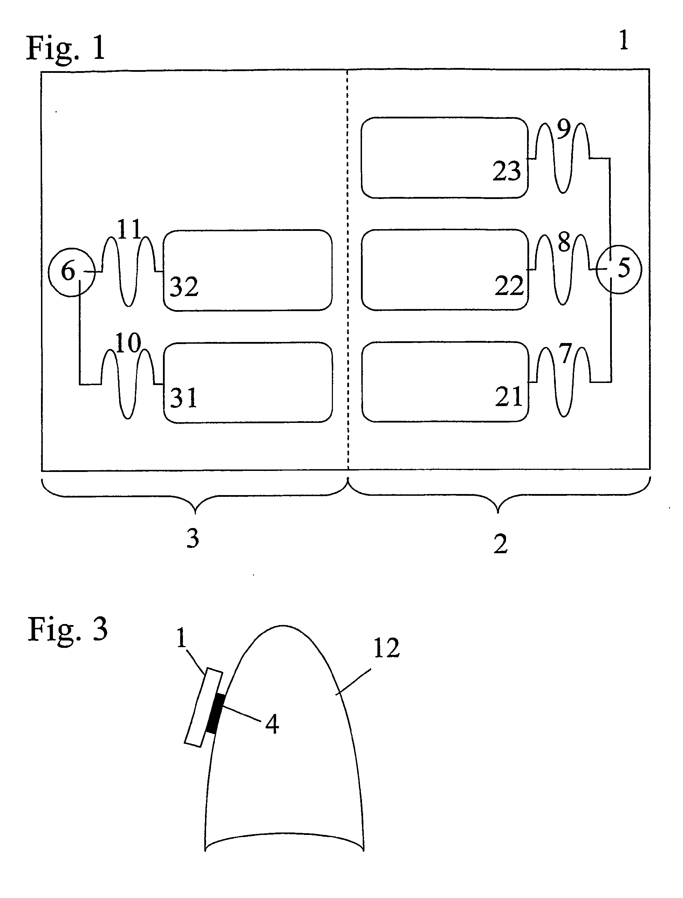

Fig. 1 zeigt ein Testelement 1 mit einer Testeinheit 2 zum Testen des Konservenbluts und eine Testeinheit 3 zum Testen des Empfängerbluts. Ein Beispiel für ein derartiges Testelement ist in der Internationalen Patentanmeldung PCT/EP 03/10590 der Anmelderin beschrieben.

Jede Testeinheit 2, 3 verfügt über einen eigenen Zugang 5, 6 für die zu testende Flüssigkeit. Hierbei handelt es sich in dem dargestellten Beispiel um Luer Lok Zugänge, an denen beispielsweise Spritzen angeschlossen werden können.

Bei der Testeinheit 2 für das Konservenblut beginnen an dem Zugang 5 drei Kanäle 7, 8, 9 durch die die zu testende Flüssigkeit, vorzugsweise Blut, zu den Reaktionskammern 21, 22, 23 gelangt. Bei der in Figur 1 dargestellten Ausführungsform enthält die erste Kammer 21 ein Anti-A-Reagenz, die zweite Kammer 22 ein Anti-B-Reagenz und die dritte Kammer 23 ein Anti-D-Reagenz. Mit Hilfe dieser Testeinheit werden die Angaben auf der Blutkonserve überprüft.

In der in Figur 1 dargestellten Ausführungsform weist die zweite Testeinheit 3 für das Testen des Bluts des Empfängers zwei Kanäle 10, 11 auf, durch die die zu testende Flüssigkeit von dem Zugang 6 zu den Reaktionskammern 31, 32 gelangt. Um mit Hilfe dieser Testeinheit 3 einen ABO-Test durchführen zu können, enthält eine Reaktionskammer 31 ein Anti-A-Reagenz und die andere Reaktionskammer ein Anti-B-Reagenz. Bei dem vorliegenden Ausführungsbeispiel sind die Kammern der beiden Testeinheiten mit gleichem Inhalt spiegelbildlich angeordnet, um ein Vergleichen der beiden Testergebnisse zu vereinfachen.
Figur 1 sieht Reaktionskammern für die Verwendung von flüssigen Reagenzien vor.

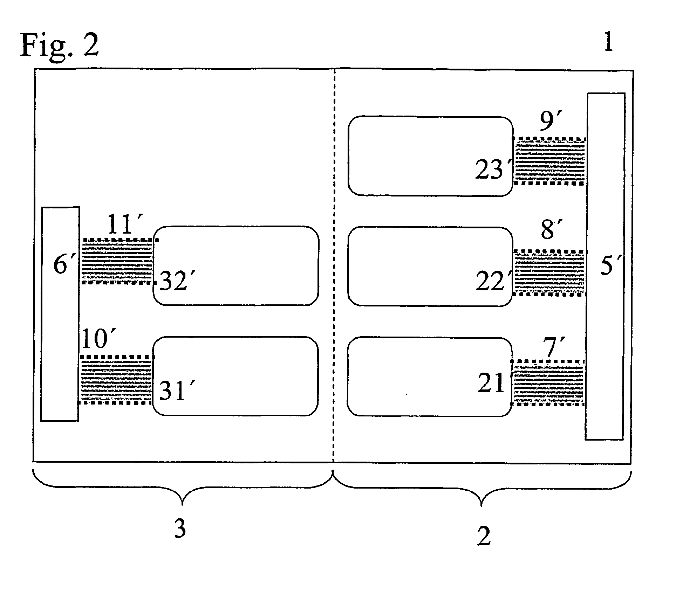

Figur 2 zeigt eine andere Ausführungsform des erfindungsgemäßen Testelements, das für immobilisierte Reagenzien geeignet ist. Das Testelement 1 ist auch hier in zwei Testeinheiten 2, 3 unterteilt. Die Testeinheiten 2, 3 verfügen über drei bzw. zwei Testfelder 21', 22', 23' bzw. 31', 32', entsprechend den Testkammern mit den gleichen Referenzzeichen ohne Beistrich in Figur 1. In diesen Testfeldern sind die zum Test erforderlichen Nachweisreagenzien in geeigneter Weise immobilisiert, also gebunden. Das Blut wird den Testfeldern 21', 22', 23' bzw. 31', 32' über Flächen 5' bzw. 6' zum Aufbringen des Blutes und über Zuleitungsflächen 7', 8', 9' bzw. 10', 11' - z.B. poröse Separationsmembranen, beispielsweise aus Nitrozellulose, in denen Blut beweglich ist - zugeführt, entsprechend den Kanälen mit entsprechenden Referenzzeichen ohne Beistrich in Figur 1. Das hier dargestellte Testelement ist derart gestaltet, dass die Zuleitungsflächen 7', 8', 9' bzw. 10', 11' in einer Ebene unterhalb der Oberfläche des Testelements 1 angeordnet sind. Wenn das Blut die Testfelder 21', 22', 23' bzw. 31', 32' erreicht, die von der Oberfläche des Testelements 1 durch eine zumindest im Bereich eines Fensters durchsichtige Schicht getrennt sind, erfolgt eine Reaktion mit den Nachweisreagenzien. Diese Reaktion kann durch den durchsichtigen Bereich der Abdeckung der Testfelder beobachtet werden. Beispiele für eine derartige Testeinheit enthält die noch unveröffentlichte deutsche Patentanmeldung mit der Anmeldenummer 103 30 982.9 vom 9. Juli 2003.
Figur 3 zeigt ein Testelement 1, das mit Hilfe des Befestigungsmittels 4 an einer Blutkonserve 12 befestigt ist, wobei das Befestigungsmittel vorzugsweise mit dem Testmittel prä-assoziiert ist.

Vorzugsweise besteht das Befestigungsmittel 4 aus einem Klebestreifen auf der Rückseite des Testelements 1. Dieser Klebestreifen kann selbstklebend sein und vor der Benutzung mit einem abziehbaren Schutzband bedeckt sein.

Das Befestigungsmittel 4 kann auch aus einer Rasteinrichtung bestehen, die in einem entsprechenden Gegenstück auf einem Blutbeutel 12 einrasten kann, derart dass sie nicht mehr oder nur mit Hilfe eines Werkzeuges - etwa eines Schlüssels - entfernbar ist.

## Patentansprüche

1. Testelement (1) für diagnostische Tests, insbesondere zum Testen von Blut vor einer Transfusion, wobei
das Testelement (1) mindestens zwei Testeinheiten (2, 3) zum Durchführen von min destens zwei Tests aufweist, und
das Testelement (1) ein Befestigungsmittel (4) zur Befestigung des Testelements (1) aufweist.

2. Testelement (1) nach dem vorhergehenden Anspruch, **dadurch gekennzeichnet, dass** das Befestigungselement (4) derart gestaltet ist, dass das Testelement (1) an einer Blutkonserve (12) befestigt werden kann.

3. Testelement (1) nach einem der vorhergehenden Ansprüche, **dadurch gekennzeichnet, dass** es sich bei dem Befestigungsmittel (4) um eine Klebefolie handelt.

4. Testelement (1) nach einem der Ansprüche 1 bis 3, **dadurch gekennzeichnet, dass** es sich bei dem Befestigungsmittel (4) um einen Kabelbinder handelt.

5. Testelement (1) nach einem der vorhergehenden Ansprüche, **dadurch gekennzeichnet, dass** bei mindestens einer der mindestens zwei Testeinheiten (2, 3) das Testergebnis mindestens 45 Tage bestehen bleibt.

6. Testelement (1) nach einem der vorhergehenden Ansprüche, **dadurch gekennzeichnet, dass** mindestens eine der mindestens zwei Testeinheiten (2, 3) derart gestaltet ist, dass nach der Durchführung des Tests keine Flüssigkeit austritt.

7. Testelement (1) nach einem der vorhergehenden Ansprüche, **dadurch gekennzeichnet, dass** mit Hilfe einer der mindestens zwei Testeinheiten (2, 3) Konservenblut für Bluttransfusionen getestet wird.

8. Testelement (1) nach dem vorhergehenden Anspruch, **dadurch gekennzeichnet, dass** die Testeinheit (2) für Konservenblut mindestens drei Testkammern (21, 22, 23) oder Testfelder (21', 22', 23') aufweist.

9. Testelement (1) nach dem vorhergehenden Anspruch, **dadurch gekennzeichnet, dass** die mindestens drei Testkammern (1, 22, 23) oder Testfelder (21', 22', 23') jeweils Anti-A, Anti-B und Anti-D Reagenzien enthalten.

10. Testelement (1) nach einem der vorhergehenden Ansprüche, **dadurch gekennzeichnet, dass** mit Hilfe der Testeinheit (3) das Blut eines Empfängers einer Bluttransfusion getestet wird.

11. Testelement 1 nach dem vorhergehenden Anspruch, **dadurch gekennzeichnet, dass** die Testeinheit (3) für das Blut eines Empfängers mindestens zwei Testkammern (31, 32) oder Testfelder (31', 32') aufweist.

12. Testelement (1) nach dem vorhergehenden Anspruch, **dadurch gekennzeichnet, dass** die mindestens zwei Testkammern (31, 32) oder Testfelder (31', 32') jeweils Anti-A und Anti-B Reagenzien enthalten.

13. Verfahren zum Testen von Blut im Rahmen der Vorbereitung und Durchführung von Bluttransfusionen, wobei das Verfahren die folgenden Schritte aufweist:
- Testen des Konservenbluts mit Hilfe der ersten Testeinheit (2) eines Testelements (1) nach einem der Ansprüche 1 bis 12;
- Befestigen des Testelements (1) auf der das Konservenblut enthaltenden Blutkonserve (12) mit Hilfe eines Befestigungsmittels (4); und
- Testen des Bluts des Empfängers mit Hilfe der zweiten Testeinheit (3) des Testelements (1).

14. Verfahren nach einem der vorhergehenden Verfahrensansprüche, **dadurch gekennzeichnet, dass** nach dem Testen des Konservenbluts und nach dem Testen des Bluts des Empfängers die Testergebnisse verglichen werden.

## Claims

1. Test element (1) for diagnostic tests, in particular for testing blood before a transfusion, wherein
the test element (1) comprises at least two test units (2, 3) for performing at least two tests, and
the test element (1) comprises a fixing means (4) for fixing the test element (1).

2. Test element (1) according to the preceding claim, **characterized in that** the fixing means (4) is formed in such a way that the test element (1) may be fixed to a blood bag (12).

3. Test element (1) according to one of the preceding claims, **characterized in that** the fixing means (4) is provided as a bonding foil.

4. Test element (1) according to one of the claims 1 to 3, **characterized in that** the fixing means (4) is provided as a cable tie.

5. Test element (1) according to one of the preceding claims, **characterized in that** in at least one of the two test units (2, 3), the test result is maintained at least 45 days.

6. Test element (1) according to one of the preceding claims, **characterized in that** at least one of the two test elements (2, 3) is formed in such a way that after the performance of the tests no fluid emerges.

7. Test element (1) according to one of the preceding claims, **characterized in that** by means of one of the at least two test elements (2, 3) bag blood for blood transfusions is tested.

8. Test element (1) according the preceding claim, **characterized in that** the test unit (2) for bag blood comprises at least three test chambers (21, 22, 23) or test fields (21', 22', 23').

9. Test element (1) according to the preceding claim, **characterized in that** the at least three test chambers (21, 22, 23) or test fields (21', 22', 23') respectively comprise anti-A, anti-B, and anti-D reagents.

10. Test element (1) according to one of the preceding claims, **characterized in that** by means of the test unit (3), the blood of a recipient of a blood transfusion is tested.

11. Test element (1) according to the preceding claim, **characterized in that** the test unit (3) for the blood of a recipient comprises at least two test chambers (31, 32) or test fields (31', 32').

12. Test element (1) according to the preceding claim, **characterized in that** the at least two test chambers (31, 32) or test fields (31', 32') respectively contain anti-A and anti-B reagents.

13. Method for testing blood during the preparation and performance of blood transfusions, wherein the method comprises the following steps:
- testing of bag blood by means of a first test unit (2) of a test element (1) according to one of the claims 1 to 12;
- fixing the test element (1) on the blood bag (12) containing the bag blood by means of a fixing means (4); and
- testing the blood of the recipient by means of the second test unit (3) of the test element (1).

14. Method according to one of the preceding method claims, **characterized in that**, after the testing of the bag blood and after the testing of the recipient blood, the test results are compared.

## Revendications

1. Élément de test (1) pour tests diagnostiques, en particulier pour tester du sang avant une transfusion, l'élément de test (1) comportant au moins deux unités de test (2, 3) pour réaliser au moins deux tests, et l'élément de test (1) comportant un moyen de fixation (4) pour fixer l'élément de test (1).

2. Élément de test (1) selon la revendication précédente, **caractérisé en ce que** l'élément de fixation (4) est conçu de façon que l'élément de test (1) puisse être fixé à une poche de sang (12).

3. Élément de test (1) selon une des revendications précédentes, **caractérisé en ce que** le moyen de fixation (4) est constitué par une pellicule adhésive.

4. Élément de test (1) selon une des revendications 1 à 3, **caractérisé en ce que** le moyen de fixation (4) est constitué par un collier de câblage.

5. Élément de test (1) selon une des revendications précédentes, **caractérisé en ce que**, dans au moins une des au moins deux unités de test (2, 3), le résultat du test est conservé pendant au moins 45 jours.

6. Élément de test (1) selon une des revendications précédentes, **caractérisé en ce qu'**au moins une des au moins deux unités de test (2, 3) est conçue de façon qu'aucun liquide n'en sorte après la réalisation du test.

7. Élément de test (1) selon une des revendications précédentes, **caractérisé en ce qu'**une des au moins deux unités de test (2, 3) permet de tester du sang de poches de sang pour transfusion sanguine.

8. Élément de test (1) selon la revendication précédente, **caractérisé en ce que** l'unité de test (2) pour poches de sang comporte au moins trois compartiments de test (21, 22, 23) ou cases de test (21', 22', 23').

9. Élément de test (1) selon la revendication précédente, **caractérisé en ce que** les au moins trois compartiments de test (21, 22, 23) ou cases de test (21', 22', 23') contiennent respectivement des réactifs anti-A, anti-B et anti-D.

10. Élément de test (1) selon une des revendications précédentes, **caractérisé en ce que** l'unité de test (3) permet de tester le sang d'un receveur d'une transfusion sanguine.

11. Élément de test 1 selon la revendication précédente, **caractérisé en ce que** l'unité de test (3) comporte au moins deux compartiments de test (31, 32) ou cases de test (31', 32') pour le sang d'un receveur.

12. Élément de test (1) selon la revendication précédente, **caractérisé en ce que** les au moins deux compartiments de test (31, 32) ou cases de test (31', 32') contiennent respectivement des réactifs anti-A et anti-B.

13. Procédé pour tester du sang dans le cadre de la préparation et de la réalisation de transfusions sanguines, le procédé comportant les étapes suivantes :
- tester le sang de poches de sang à l'aide de la première unité de test (2) d'un élément de test (1) selon une des revendications 1 à 12 ;
- fixer l'élément de test (1) à l'aide d'un moyen de fixation (4) à la poche de sang (12) contenant le sang de la poche de sang, et
- tester le sang du receveur à l'aide de la seconde unité de test (3) de l'élément de test (1).

14. Procédé selon une des revendications de procédé précédentes, **caractérisé en ce qu'**après le test du sang de la poche de sang et après le test du sang du receveur les résultats de test sont comparés.
